# EUROPEAN PATENT APPLICATION

(11) **EP 4 439 775 A1**
(43) Date of publication of application: **02.10.2024**
(21) Application number: 23275046.3
(22) Date of filing: 28.03.2023
(51) Int. Cl.: H01M 10/42, H01M 10/48, H01M 6/50

(54) **GAS DETECTION IN BATTERIES**

(71) Applicant: Kidde Graviner Limited, Shirley, Solihull B90 4SS (GB)
(72) Inventor: DUNSTER, Robert G., Burnham, SL1 6ER (GB); PORTERFIELD, John W., Rolesville, 27571 (US); ROUSE, Albert C., Prior Lake, 55372 (US); LEWIS, John, Elm City, 27822 (US); STEIN, Barret, Raleigh, 27616 (US)
(74) Representative: Dehns

(57) **Abstract**

A method of detecting a cell venting event in a battery pack, comprising: determining a rate of increase of the concentration of a gas in the battery pack with respect to time; providing an indication that the determined rate of increase of the concentration of the gas has exceeded a first preset rate of increase threshold value.

## Description

### TECHNICAL FIELD

The present disclosure relates to methods and systems for gas detection in batteries, for example in lithium-ion batteries. The gas detection methods and systems may be employed in fire suppression systems for said batteries.

### BACKGROUND

A battery pack typically includes many individual battery cells arranged in series and in parallel. The individual cells may rupture due to unfavorable external or internal battery conditions. A failing battery cell may leak gaseous matter into the surrounding battery pack. In the case of a gas leak, a battery may be subject to a fire event, and in extreme cases, an explosion, which may propagate to other nearby battery cells. This can cause thermal runaway within the whole battery pack, resulting in large-scale fire event and destruction of the battery. When such an event occurs in a battery pack in a vehicle, such as an airplane, the consequences can be extremely dangerous. Attempts have been made to predict potential fire events by monitoring the concentration of gas within battery packs. When the measured concentration of the gas rises above a particular predetermined threshold, a cell venting event is indicated and an alarm signal is generated. The alarm signal can then be used to automatically or manually prompt fire avoidance or suppression actions, such as delivery of a fire suppressant material into the battery pack, depowering the battery pack, disposal of the battery pack and/or evacuation of a vehicle in which the battery pack may be located. If the detection of the venting event and subsequent fire avoidance or suppression action are carried out fast enough, thermal runaway and large-scale fire or battery destruction can be avoided. One of the problems, however, with utilizing the measurement of gas concentration in the battery pack as an indicator of a cell venting event is that the overall concentration of gas may rise steadily past the predetermined threshold due to other, normally occurring, non-dangerous factors. Continuous cycling and heavy loads on batteries can cause a degree of electrolyte degradation, resulting in the generation of gas within the cells. This gas inevitably dissipates into the battery pack. This is called natural gas generation, and is not necessarily indicative of an imminent fire event within the battery. Such natural gas generation may cause the measured concentration of gas within the battery pack to exceed the predetermined threshold used in the aforementioned cell venting detection methods, thereby giving rise to a false indication of a cell venting event. This may result in unnecessary fire avoidance or suppression actions being taken, which may result in unnecessary disposal of the battery or evacuation of a vehicle in which the battery pack may be held. The present disclosure provides a method and system for detecting cell venting events in battery backs designed to overcome the problems associated with the state of the art solutions.

### SUMMARY

According to the disclosure, there is provided a method of detecting a cell venting event in a battery pack, comprising: determining a rate of increase of the concentration of a gas in the battery pack with respect to time; providing an indication that the determined rate of increase of the concentration of the gas has exceeded a first preset rate of increase threshold value.

Also provided is a system, comprising: a battery pack, comprising one or more cells; a gas sensor; a controller; wherein the gas sensor is configured to measure a concentration of a gas in the battery pack; wherein the controller is configured to: determine a rate of increase of the concentration of the gas with respect to time and provide an indication that the determined rate of increase of the concentration of the gas has exceeded a first preset rate of increase threshold value

### BRIEF DESCRIPTION OF THE DRAWINGS

The figures show selected embodiments of the method and system described herein. It will however be appreciated that the disclosure is not limited to any of the embodiments illustrated in the figures and that the scope of protection is defined by the claims.
Figure 1A illustrates a detection system and a battery pack according to the disclosure.
Figure 1B illustrates a method of detecting a cell venting event in a battery pack according to the disclosure.
Figure 2A is a graph illustrating two cell venting events in a battery pack and a rate of rise measurement of the concentration of gas in the battery pack.
Figure 2B is a graph illustrating multiple rate of rise calculations after a cell venting event.
Figure 3 illustrates a method of detecting a cell venting event in a battery pack according to the disclosure.
Figure 4 is a graph illustrating multiple rate of rise calculations after a cell venting event and in the presence of electromagnetic interference.
Figure 5 is a graph illustrating rate of rise calculations and comparisons thereof with two different rate of rise threshold values after a cell venting event.

### DETAILED DESCRIPTION

Various embodiments of the gas detection method and system are described in detail below. The features of each embodiment can be combined and/or substituted with features of any other embodiment, unless explicitly disclosed otherwise.

As discussed in the Background section above, the rapid release of gas from the vent of a single cell (failure of the cell) within a battery pack is enough to initiate thermal runaway and/or a fire event within a battery pack. It is therefore important to provide the necessary rapid detection of a failed battery cell at the very early stages after a cell has vented. It has been shown that, if detected early enough, removing the load from a battery pack where a single cell has vented can prevent thermal runaway. Additionally, active delivery of suppressing agent into the battery pack(s) at this early stage has shown improved cooling, resulting in either prevention or mitigation of the effects of thermal runaway. Research has shown that continuous cycling and heavy loads on batteries can cause a degree of electrolyte degradation resulting in the generation of gases within a cell structure. Since the battery cells are not designed to be sealed pressure vessels, a portion of this gas may inevitably dissipate into the battery pack. Such natural gas generation is not typically cause for concern, and should not by itself trigger thermal runaway.

In order to provide rapid indication of a failed battery cell and therefore enable mitigation of resultant thermal runaway, known systems and methods employ monitoring of the concentration of gas within the battery pack. Once the concentration of the gas reaches a predetermined threshold, an alarm signal is generated, and appropriate fire event/thermal runaway prevention actions may be performed in response thereto.

One of the problems with such gas detection systems and methods is that natural gas generation may result in concentrations of gas within the battery pack exceeding the required thresholds to trigger an alarm signal, or generate a general indication that said thresholds have been exceeded. This would be undesirable, since measures may consequently be taken to suppress non-existent thermal runaway or fire events, needlessly hindering the short- or long-term operation of the battery pack. The present challenge, therefore, in rapid detection of battery failure, is to distinguish between gas generation during normal cycling and a cell venting event which requires immediate preventative action, without compromising on detection speed or reliability.

The present disclosure, provides an improved system and method for gas detection in battery packs, which may be, but are not necessarily, lithium-ion battery packs, capable of accurately and rapidly detecting a venting event of a battery cell, which may be an indicator of imminent thermal runaway or fire.

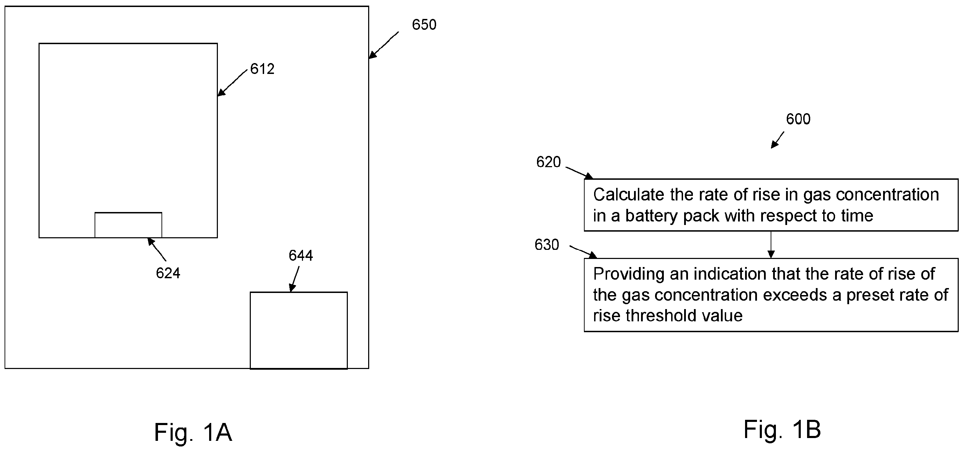

Figure 1A illustrates a gas detection system 650 according to the present disclosure. The system 650 includes a battery pack 612, a gas sensor 624 and a controller 644. The battery pack 612 may be a lithium-ion, or similar, battery pack. The battery pack 612 may be comprised of a collection of individual battery cells, which may be combined in series or parallel in order to tune or raise the voltage and capacity of the battery pack 612. The gas sensor 624 is configured to measure a concentration of a gas in the battery pack 612. Typically, as may be the case with the gas sensor(s) 624 described herein, such gas sensors 624 may be configured to output a voltage signal which is indicative of the sensed concentration of gas. However, other types of gas sensor may also be utilised. Together, the gas sensor 624 and the controller may be configured to (where each step is either performed by the gas sensor 624 or controller 644, in any combination): calculate a rate of rise, i.e. rate of increase with respect to time, of the concentration of a gas in the battery pack 612 over a preset length of time; compare the calculated rate of rise of the gas concentration with a preset rate of rise threshold value; and if the calculated rate of rise exceeds said rate of rise threshold value, provide an indication thereof. The controller 644 may be further configured to generate an alarm signal and/or take measures to mitigate thermal runaway or fire upon provision of this indication. The measures to mitigate thermal runaway or fire can be any of those described in this application.

Figure 1B illustrates a method 600 according to the present disclosure, which may be used with the system of Figure 1A. In this method, the rate of rise, i.e. the rate of increase with respect to time, of the concentration of the gas in the battery pack is determined. If the determined rate of rise of the concentration of the gas exceeds a preset rate of rise threshold value, an indication thereof is provided. In response to this indication, an alarm signal may be generated or some other response or action may follow. By utilising a determination of the rate of rise of a gas concentration in the battery pack as opposed to a simple gas concentration measurement to detect a cell rupture or venting event, a situation in which an indication of a cell venting event is provided due to natural gas generation in the battery, in absence of such a cell rupture or venting event, is avoided. Natural gas generation typically only causes a gradual increase in concentration of a gas in the battery pack. Therefore, the rate of rise of gas due to natural gas generation will not trigger provision of an indication of a cell venting event. In the method 600, in order to determine the rate of rise of the concentration of gas, measurements may first be made of the gas concentration at a first point in time and a second point in time, a preset amount of time after the first point in time. These measurements may be taken by one or more gas sensors 624 and said gas sensors 624 may output a voltage signal indicative of the sensed concentration of gas, thereby enabling measurements of the gas concentration. The rate of rise may then be calculated using the gas concentration measurements at the first and second points in time and the time interval between the first and second points in time.

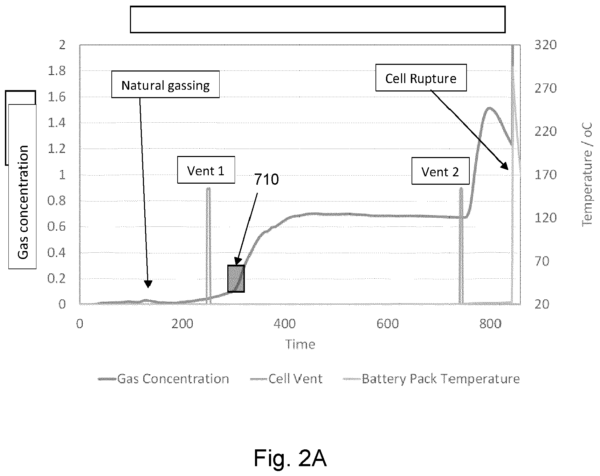

Figure 2A shows a graph showing sensed gas concentration in a battery pack with respect to time. The graph also includes lines illustrating two cell rupture, or venting, events, "Vent 1" and "Vent 2", and a line representative of the battery pack temperature. As can be seen from the figure, around the time the first venting event, "Vent 1", occurs, the gas concentration increases rapidly. Similarly, around the time of the second venting event, "Vent 2", the gas concentration further increases rapidly. A short time after the second venting event, both the gas concentration and battery pack temperature increase very rapidly. This sequence of events is indicative of thermal runaway in the battery pack.

When such a sequence of events occurs when the system and/or method of the present disclosure is utilised, the initial rapid increase in gas concentration caused by the first venting event may cause generation of an indication that the rate of rise has exceeded a threshold, which may in turn cause generation of an alarm signal. This is because the rate of rise of the concentration in the gas during the time period indicated by reference numeral 710 would be sufficiently high, i.e. above a preset rate of rise threshold value, to generate said indication in the associated gas sensor 624 and/or controller 644. A rectangle is overlaid onto the graph at point 710. The width of the rectangle, i.e. the extension thereof along the "Time" axis corresponds to the length of time over which the rate of rise is measured. The height of the rectangle, i.e. the extension of the rectangle in the "Concentration" axis corresponds to the magnitude of the increase in gas concentration in the battery pack in the time interval determined by the width of the rectangle. It will be appreciated that the rate of rise of the concentration of gas may be calculated by dividing the magnitude of the increase in gas concentration over the length of time elapsed during said time interval.

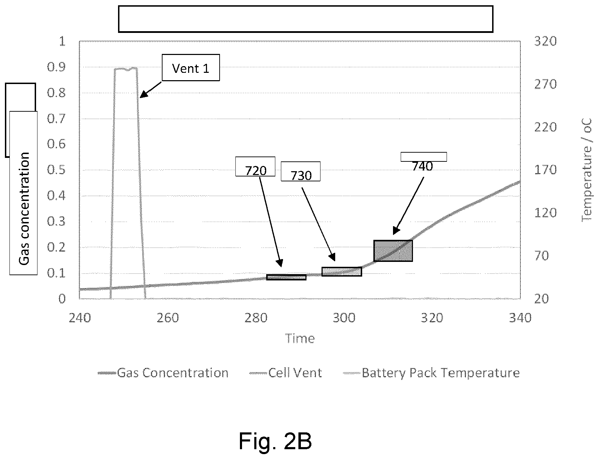

Figure 2B shows a graph similar to the graph of Figure 2A. Three rectangles 720, 730 and 740 are overlaid onto the graph and represent three consecutive calculations of the rate of rise of the gas concentration in a battery pack, each over the same length of time. The rates of rise of gas concentration over the elapsed length of time in the first two measurements, 720 and 730, do not exceed a preset rate of rise threshold value, and no indication is provided of said preset rate of rise threshold value being exceeded in response. However, the rate of rise of the gas concentration during the time interval indicated by reference numeral 740 exceeds the preset rate of rise threshold value and triggers generation of such an indication, which may in turn trigger an alarm signal and/or a fire suppression action.

Whilst specific lengths of time have been illustrated with the overlaid rectangles in Figures 2A and 2B, it will be appreciated that the actual length of time may be selected based on one or more parameters such as battery pack size, structure or type. Equally, the rate of rise threshold value may be selected to meet the needs of a specific battery system.

One way in which the method 600 of the present disclosure may be implemented is by continually measuring the gas concentration in the battery pack, storing these measurements with their associated timestamps and continually making calculations of the rate of rise of the gas concentration using gas concentration measurements between time points, the length of time between time points corresponding to a preselected length of time. The frequency at which this is performed may be chosen to find a balance between avoiding excessive computation/energy consumption and ensuring the reliability and speed of detecting cell venting events. The advantage of this method is that a venting event can be detected based on the rate of rise of the concentration of gas in the battery pack alone, without taking into account the actual magnitude of gas concentration in the battery pack. This may be useful when the expected values for the magnitude of the concentration of the gas in the battery due to natural gas generation alone and/or due to dangerous battery failure are not known.

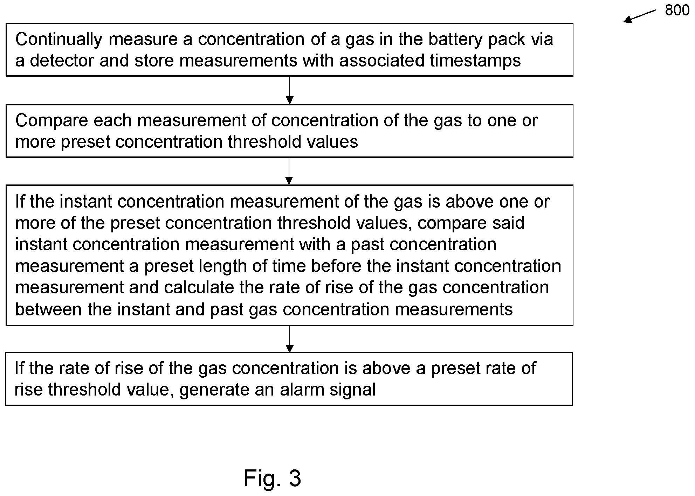

Figure 3 illustrates a method 800, which is another example of how the method 600 may be carried out. In this method 800, the concentration of a gas in a battery pack is continually measured. The measurements are stored with their associated timestamps. Each measurement is compared to one or more preset concentration threshold values. If a measurement exceeds one or more of the preset concentration threshold values, a calculation of the rate of rise of the gas concentration over a preset length of time is made using the instant concentration measurement value and a previously stored concentration measurement value, measured at a point in time the preset length of time before to the instant measurement. In this way, a calculation of the rate of rise of the gas concentration is made retrospectively, based on the preset length of time leading up to the measurement exceeding the concentration threshold value. One advantage of this method is that rate of rise calculations are only made once a preset concentration threshold value is met, which may reduce computational burden.

In some embodiments, including any of the embodiments mentioned in this application, the preset rate of rise threshold value may be automatically adjusted once a preset gas concentration threshold value is reached. For example, when a particular overall gas concentration in the battery pack exceeds a certain value, the preset rate of rise threshold value, which when exceeded causes provision of an indication thereof, can be raised or lowered. Similarly, the preset length of time over which the rate of rise is calculated may also be automatically adjusted, i.e. lengthened or shortened, in response to a particular gas concentration being reached or exceeded. It is also anticipated that the frequency of gas concentration measurements and/or frequency of rate of rise calculations may be raised or lowered in response to a particular gas concentration in the battery pack being reached or exceeded. Automatic adjustment of the aforementioned parameters upon a particular gas concentration threshold value being met or reached can increase the reliability of detecting a cell venting event, which may be compromised when greater concentrations of gas are present in the battery pack.

In some embodiments, including any of the embodiments mentioned in this application, an indication may be provided of a preset rate of rise threshold value being exceeded as well as an indication being provided of a preset critical concentration threshold value being exceeded. For example, when the concentration of gas in a battery pack is below a preset critical concentration threshold, an indication may be provided of the rate of rise of the gas concentration exceeding a preset rate of rise threshold value. In addition, an indication of the gas concentration exceeding the preset critical concentration threshold value may also be provided. This indication will be provided irrespective of the time taken for said critical concentration threshold value to be exceeded. Either the indication of the preset rate of rise threshold value being exceeded or the indication of the preset critical concentration value being exceeded may generate an alarm signal. The two indications may additionally or alternatively cause provision of two distinct, respective, alarm signals. The features of this embodiment, i.e. the provision of an indication of the gas concentration exceeding a critical concentration threshold, may be combined with any of the other disclosed methods. For example, when this embodiment is applied to the method 800 illustrated in Figure 3, two concentration threshold values may be set, a first concentration threshold value and a critical concentration threshold value. When the concentration of gas is under the first concentration threshold value, the concentration of gas is measured, but no rate of rise calculations are performed. When the first concentration threshold value (the same concentration threshold value as originally defined in method 800) is exceeded, calculation of the rate of rise of the gas concentration is performed and an indication of the rate of rise of the gas concentration exceeding a preset rate of rise threshold value is provided. In addition, if the gas concentration value exceeds the critical concentration threshold value, an indication thereof will provided, irrespective of whether the rate of rise of the gas concentration has exceeded the preset rate of rise threshold value. Either indication may induce an alarm signal. Each indication may induce its own respective alarm signal. This embodiment provides the advantage that an alarm signal can be generated in the event dangerous concentrations of gas in the battery pack are reached, even if the rate of rise of the gas concentration was not sufficiently high to trigger an alarm itself.

In practice, battery packs such as those described in relation to any of the embodiments of this application may be installed in systems comprising one or more high energy components. Electromagnetic compatibility, or EMC, becomes a significant consideration when detecting gas concentrations in battery packs placed in or around such high energy components, particularly when the components are not adequately earthed or packaged. Electromagnetic interference from nearby high energy components can cause erroneous readings in gas concentration sensors in the battery pack. Typical gas sensors output a voltage signal indicative of the concentration of gas in the battery pack. Electromagnetic interference can cause occasional spikes in the voltage output of these gas sensors, erroneously indicating a sudden spike in gas concentration in the battery pack. These spikes in voltage are usually very high and often do not last for very long.

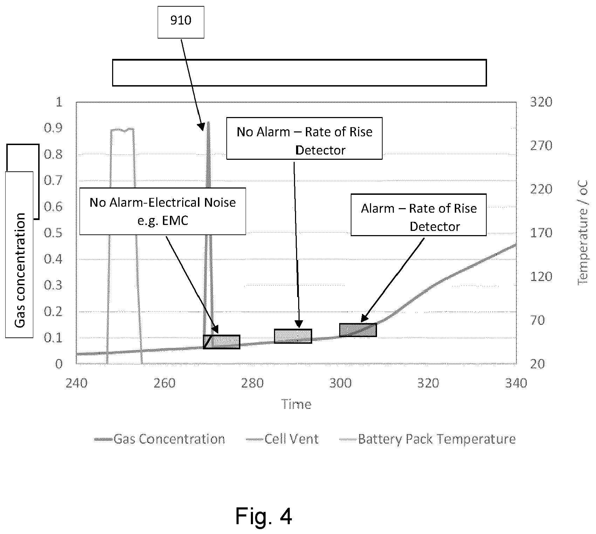

Figure 4 illustrates a graph similar to that shown in Figure 2B. However, in the graph illustrated in Figure 4, a spike 910 is present in the voltage signal indicative of the gas concentration in the battery pack 612 due to electromagnetic interference experienced by the gas sensor 624.

In some embodiments, including any of the embodiments described in this application, an indication of the rate of rise of the gas concentration exceeding the preset rate of rise threshold value will not be provided unless the concentration of gas and/or the rate of rise of the gas concentration exceed, respectively, a particular preset concentration threshold value or the preset rate of rise threshold value for a preset length of time. This functionality provides the advantage that voltage spikes due to electromagnetic interference, such that that indicated by reference numeral 910 in Figure 4 do not result in provision of an indication of the gas concentration exceeding the preset rate of rise threshold, which is itself indicative of a cell venting event. This will also avoid an alarm signal being generated in response thereto. As mentioned previously, erroneous generation of an alarm signal in absence of an actual cell venting event is undesirable, and can lead to unnecessary mitigation measures being taken.

In addition to the above, an upper rate of rise threshold value can be set. In any of the embodiments described herein, an indication of the gas concentration exceeding the preset rate of rise threshold may not be provided if the measured rate of rise of the gas concentration is above this upper rate of rise threshold value. It can be appreciated that this will reduce the risk of erroneous generation of an alarm signal due to an electrical spike caused by electromagnetic interference, which typically causes a very sharp increase in the voltage signal from a gas sensor.

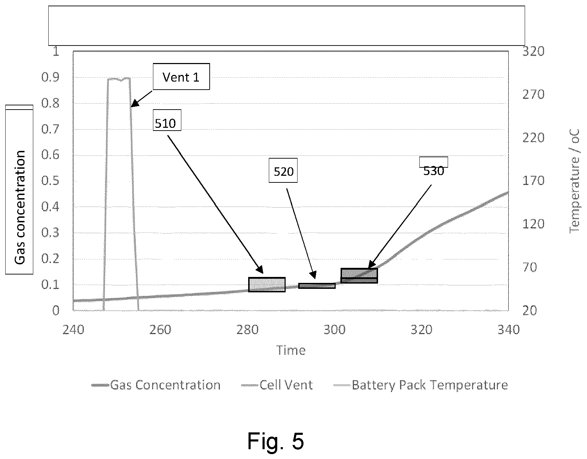

Figure 5 illustrates a graph similar to that shown in Figure 2B. The graph shows the change in gas concentration with respect to time after a cell venting event "Vent 1" and subsequent sequential measurements of the rate of rise of the gas concentration after said cell venting event. The rate of rise measurements are depicted using rectangles as in the previous figures. As can be seen in the figure, two different size rectangles are overlaid. The rectangle denoted by reference numeral 510 represents a comparison of the measured rate of rise of the gas concentration against a significant level rate of rise threshold value. Since the rate of rise of the gas concentration over the time period spanned by the rectangle at 510 does not meet or exceed the value required to trigger a positive indication that the rate of rise has exceeded the significant rate of rise threshold value, no indication of a significant level rate of rise is produced. The rectangle denoted by reference numeral 520 illustrates a comparison of the measured rate of rise of the gas concentration over this time period against a trace level rate of rise threshold. As can be seen, the rate of rise of the gas concentration across the rectangle at 520 is almost the same as that across the rectangle at 510. However, due to the trace level rate of rise threshold being sufficiently lower than the significant level rate of rise threshold, the measured rate of rise of the gas concentration across the rectangle at 520 is sufficient (meeting or exceeding the trace level rate of rise threshold value) to cause production of an indication of a trace level rate of rise. Reference numeral 530 represents a theoretical simultaneous comparison of the measured rate of rise of the gas concentration with the trace level rate of rise threshold and the significant level rate of rise threshold, where the measured rate of rise threshold exceeds the significant level rate of rise threshold value. In such a situation, since the significant level rate of rise threshold value is greater than the trace level rate of rise threshold value, indications of both a trace level and significant level rate of rise are both produced.

In any of the embodiments described throughout this disclosure, the rate of rise threshold value(s) may be replaced with this dual rate of rise threshold, i.e. with two threshold values, a trace level rate of rise threshold value and a significant rate of rise threshold value. The use of two different threshold values in place of one enables more detailed monitoring of the development of gas concentration in a battery pack and therethrough better informed decision making with regards to fire or thermal runaway prevention actions.

Although this disclosure has been described in terms of preferred examples, it should be understood that these examples are illustrative only and that the claims are not limited to those examples. Those skilled in the art will be able to make modifications and alternatives in view of the disclosure which are contemplated as falling within the scope of the appended claims.

## Claims

1. A method of detecting a cell venting event in a battery pack, comprising:
determining a rate of increase of the concentration of a gas in the battery pack with respect to time;
providing an indication that the determined rate of increase of the concentration of the gas has exceeded a first preset rate of increase threshold value.

2. The method of claim 1, further comprising:
measuring the concentration of the gas using a gas sensor; and optionally wherein the gas sensor outputs a voltage signal indicative of the concentration of the gas.

3. The method of claim 1 or 2, further comprising:
calculating the rate of increase of the concentration of the gas using a first measured value of the concentration of the gas at a first point in time, and a second measured value of the concentration of gas at a second point in time, said second point in time being a first preset length of time after the first point in time.

4. The method of any preceding claim, further comprising:
generating an alarm signal in response to the provision of the indication that the measured rate of increase of the concentration of the gas has exceeded the first preset rate of increase threshold value.

5. The method of claim 4, further comprising:
in response to the generation of the alarm signal, taking one or more fire prevention measures; wherein said one or more fire prevention measures optionally include: disposing of the battery pack, depowering the battery pack, removing the battery pack from a vehicle and/or introducing a fire suppressant substance into the battery pack.

6. The method of claim 3, 4 or 5, wherein said the step of determining a rate of increase of the concentration of a gas in the battery pack with respect to time is only performed after the concentration of the gas exceeds a first preset gas concentration value.

7. The method of any preceding claim, further comprising:
providing an indication that the concentration of the gas exceeds a preset critical concentration threshold value; and optionally generating a critical alarm signal in response to said indication of the concentration of gas exceeding the preset critical concentration threshold value.

8. The method of any of any of claims 4 to 7, wherein
said step of generating the alarm signal only occurs in the event that the rate of increase of the concentration of the gas is sustained above the first preset rate of increase threshold for a second preset length of time.

9. The method of any of claims 4 to 8, wherein
said step of generating the alarm signal only occurs in the event that the concentration of the gas is sustained above a second preset gas concentration value for a third preset length of time.

10. The method of any of claims 4 to 9, wherein
if the determined rate of increase of the concentration of the gas exceeds a second preset rate of increase threshold value, no alarm signal is generated in response to said calculated rate of increase.

11. The method of any of claims 3 to 10, further comprising:
if the concentration of the gas exceeds a first preset saturation threshold value, raising or lowering the first preset length of time; and/or raising or lowering the first preset rate of increase threshold value.

12. The method of any of claims 6 to 11, wherein
if the concentration of the gas exceeds a second preset saturation threshold value, the first preset gas concentration value is raised or lowered.

13. The method of any preceding claim, wherein the battery pack is a lithium-ion battery pack.

14. A system (650), comprising:
a battery pack (612), comprising one or more cells;
a gas sensor (624);
a controller (644);
wherein the gas sensor (624) is configured to measure a concentration of a gas in the battery pack;
wherein the controller (644) is configured to:
determine a rate of increase of the concentration of the gas with respect to time and provide an indication that the determined rate of increase of the concentration of the gas has exceeded a first preset rate of increase threshold value.

15. A fire suppression system for a battery pack, comprising
the system of claim 14;
means for suppressing a fire event in the battery pack;
wherein the means for suppressing a fire event is configured to be deployed in response to the provision, by the controller, of the indication the determined rate of increase of the concentration of the gas has exceeded the first preset rate of increase threshold value.
